# EUROPÄISCHE PATENTSCHRIFT

(11) **EP 0 621 286 B1**
(45) Veröffentlichungstag und Bekanntmachung des Hinweises auf die Patenterteilung: **09.10.1996**
(21) Anmeldenummer: 94103800.2
(22) Anmeldetag: 11.03.1994
(51) Int. Cl.: C08B 15/05, C08B 31/00, C07H 23/00

(54) **Verfahren zur Silylierung von Kohlenhydraten und Verwendung der silylierten Kohlenhydrate**
Process for the silylation of carbohydrates and use of the silylated carbohydrates
Procédé de silylation d'hydrates de carbone et utilisation des hydrates de carbone silylatés

(30) Priorität: 23.03.1993 DE 4309297
(43) Veröffentlichungstag der Anmeldung: 26.10.1994
(73) Patentinhaber: RHONE-POULENC RHODIA AKTIENGESELLSCHAFT, 79108 Freiburg (DE)
(72) Erfinder: Wagner, Thomas, Dipl.-Chem., D-57482 Wenden (DE); Mormann, Werner, Prof. Dr., D-57223 Kreuztal (DE)
(74) Vertreter: Hagemann, Heinrich, Dr.rer.nat., Dipl.-Chem.

(56) Entgegenhaltungen:
- EP-A- 0 562 378
- DD-A- 263 416
- DE-A- 3 104 531
- CARBOHYDRATE RESEARCH Bd. 31 , 1973 , AMSTERDAM Seiten 407 - 409 R. E. HARMON ET AL. 'New procedure for preparing trimethylsilyl derivatives for polysaccharides'
- DAS PAPIER Bd. 35, Nr. 12 , 1981 , DARMSTADT Seiten 547 - 554 G. GREBER ET AL. 'Silylderivate der Cellulose'
- JOURNAL OF APPLIED POLYMER SCIENCE Bd. 26 , 1981 Seiten 3827 - 3836 GEOFFREY K. COOPER ET AL. 'Trimethylsilyl Cellulose as Precursor to Regenerated Cellulose Fiber'

## Beschreibung

Die Erfindung betrifft ein Verfahren zur Silylierung von Kohlenhydraten in flüssigen Stickstoffverbindungen und verschiedene Verwendungsmöglichkeiten der danach erhaltenen silylierten Kohlenhydrate.

Durch Silylierung von Kohlenhydraten lassen sich deren chemische und physikalische Eigenschaften drastisch ändern. Die Silylierung erfolgt durch Bindung von Trialkylsilylgruppen an die Hydroxylgruppen der Kohlenstoffatome 2, 3 und 6 der Anhydroglucoseeinheit unter Bildung von Si-O-Bindungen. Trimethylsilylderivate von Kohlenhydraten mit einem durchschnittlichen Substitutionsgrad von etwa 2,5 sind bekannt (vgl. u.a. H.A. Schuyten et al. in J. Am. Chem. Soc**. 70**, 1919 ff (1948)). Das gebräuchlichste Silylierungsmittel ist das leicht zugängliche Chlortrimethylsilan. So wurde Cellulose nach zahlreichen Arbeiten mit Chlortrimethylsilan in Pyridin als Reaktionsmedium zu Trimethylsilylcellulose umgesetzt. Das Pyridin diente gleichzeitig als Quellmittel und auch als Säureakzeptor der heterogen verlaufenden Reaktion.

In J. Am. Chem. Soc**. 70**, 1919 ff (1948) wird erstmals die Darstellung eines Silylderivats der Cellulose beschrieben. Danach wurden unter heterogenen Bedingungen durch Umsetzung von Cellulose (Baumwoll-Linters) sowie Celluloseacetat mit Chlortrimethylsilan in Pyridin unter Rückfluß Trimethylsilyl-Derivate mit maximal 2,75 Trimethylsilyl-Gruppen pro Anhydroglucoseeinheit der Cellulose erhalten. Diese Trimethylsilylcellulose war bei Raumtemperatur an der Luft stabil. Sie zersetzte sich bei höheren Temperaturen oder bei Anwesenheit von Luftfeuchtigkeit. In siedendem Wasser oder in verdünnten Säuren und Basen hydrolysierte sie sehr schnell. Bei teilweise acetylierter Cellulose reagierten ausschließlich die noch freien Hydroxylgruppen, wobei sich ein Gesamtsubstitutionsgrad von nahezu 3 ergab.

Nach J. Polym. Sci. Part A-1 **7**, 1947 ff (1969) wird durch 7-stündige Reaktion (160-170°C) von Cellulose mit Bis(trimethylsilyl)-acetamid als Silyierungsreagenz in N-Methylpyrrolidon und Xylol als Lösungsmittel Trimethylsilylcellulose mit ebenfalls 2,7 Trimethylsilylgruppen pro Anhydroglycoseeinheit erhalten. Durch 10-stündige Behandlung dieser Trimethylsilylcellulose mit siedendem Wasser wurden nur 50% der Trimethylsilylgruppen hydrolytisch abgespalten. Diese überraschend hohe Hydrolysebeständigkeit wird auf sterische Faktoren zurückgeführt. Neben Trimethylcellulose wurden weitere Triorganosilylcellulosen hergestellt, die aufgrund ihrer niedrigen relativen Dielektrizitätskonstanten in erster Linie als Elektroisolationsmaterial Interesse fanden.

In Ind. Eng. Chem. **45**, 2542 ff (1953) wird erstmals die Silylierung von Stärke beschrieben. Danach wird Maisstärke mit Chlortrimethylsilan als Silylierungsreagenz in Formamid umgesetzt und ein Produkt mit einem Substitutionsgrad bis zu 2,0 erhalten.

In Makromol. Chem. **21**, 59 ff (1956) wird die Übertragung der Silylierung von Glucose auf Pektin beschrieben. Es wurden mit verschiedenen Alkyl- und Arylchlorsilanen Derivate mit einem Substitutionsgrad unter 1 erhalten.

In Makromol.Chem. **126**, 139 ff (1969) wird die Synthese von 2,3,6-Tris-O-trimethylsilyl- amylose und -cellulose durch Umsetzung der Kohlenhydrate in der Schmelze (170°C) mit N-Trimethylsilylacetamid beschrieben. Die Silylierung nativer Cellulose-Linters lieferte im Gegensatz zu aus Acetylcellulose regenerierter Cellulose lediglich Substitutionsgrade von 2,4.

Nach Makromol. Chem. **120**, 87 ff (1968) gelingt eine Persilylierung von Amylose und Cellulose durch heterogene Umsetzung der entsprechenden Kohlenhydrate für 3 Stunden bei 15°C in Pyridin mit Chlortrimethylsilan. Bei nativer Cellulose (Baumwoll-Linters) mußte die Umsetzung wiederholt werden, da im Gegensatz zu aus Cuoxam (Schweizers Reagens) umgefällter Cellulose noch freie Hydroxylgruppen vorhanden waren. Eine vollständige Substitution der verzweigten Polysaccharide Dextran und Amylopektin wurde nicht erreicht.

Da bei der Silylierung mit Chlortrimethylsilan in Gegenwart von tertiären Aminen oder Ammoniak stets Hydrochlorid als Nebenproudkt bzw. Verunreinigung anfällt, verwendeten Harmon et. al. (vgl. Carbohyd. Res. **31**, 407 ff (1973) und "Die Stärke" **25**, 429 ff (1973)) Hexamethyldisilazan als Silylierungsmittel. Damit stellten sie in Pyridin bzw. in Formamid, einem stark polaren organischen Lösungsmittel, bei 70°C Trimethylsilylderivate von Stärke, Amylose, Amylopektin, Glykogen, Chitin, Dextrinen, Pektin und Cellulose dar. Das Trimethylsilylderivat von Polysacchariden wird dabei beispielsweise dadurch ausgefällt, indem dessen Lösung in Formamid in wasserfreies Aceton gegossen wird. Vollständig silyliert wurden nur Cellulose und niedermolekulare Dextrine. Selbst die ebenfalls unverzweigte Amylose wurde nur bis zu einem Substitutionsgrad von 2,2 silyliert. Das Trimethylsilylderivat des vezweigten Polysaccharids Amylopektin enthielt im Vergleich dazu nur 0,9 Trimethylsilylgruppen pro Anhydroglucoseeinheit.

Alle oben beschriebenen Arbeiten zur Silylierung von Kohlenhydraten hatten das vorrangige Ziel, Trimethylsilylderivate mit hohem Substitutionsgrad, die in organischen Lösungsmitteln löslich sind und sich als hydrophobe Filme, Papiere oder Membrane einsetzen lassen, zu gewinnen.

Um die begrenzten Einflüsse, die sich aus der übermolekularen Stuktur der Kohlenhydrate und einer daraus resultierenden mehrphasigen Reaktionsführung ergeben, zu umgehen, wurde für Cellulose auch eine Reihe von nicht-wäßrigen Lösungsmitteln verwendet. So modifizierte man Cellulose durch Behandlung mit derivatisierenden Systemen, wie Distickstofftetroxid/N,N-Dimethylformamid (vgl. R.G. Schweiger, Tappi **57**, 86 ff (1974)) oder Dimethylsulfoxid/Paraformaldehyd (vgl. N.Shirashi et al., Sen'i Gakkaishi **35**, 466 ff (1979)), unter Bildung löslicher instabiler Derivate (Nitrite bzw. Hydroxymethylether) und durch anschließende Folgereaktionen an den Hydroxylgruppen bzw. an den intermediär entstandenen funktionellen Gruppen, die wieder abgespaltet wurden. Dabei wurden Trimethylsilylderivate von Cellulose mit N,O-Bis(trimethylsilyl)trifluoracetamid im Lösungsmittelsystem Dimethylsulfoxid/Paraformaldehyd dargestellt. Es wurden trotz homogener Reaktionsführung nur Substitutionsgrade von 2,4 erreicht (vgl. N.Shirashi et al., Sen'i Gakkaishi **35**, 466 ff (1979)).

Eine weitere Möglichkeit für die homogene Modifizierung von Cellulose stellt die Auflösung in speziellen nicht-derivatisierenden Lösungsmittelsystemen, wie N,N-Dimethylacetamid/Lithiumchlorid (vgl. W. Schempp et al., Das Papier **38**, 607 ff (1984)) oder N-Methyl-morpholin-N-oxid (vgl. J.F. Kennedy et al., Cellulose, Ellis Horwood Ltd., Chichester 1990), mit anschließender Umsetzung an den Hydroxylgruppen dar. "Das Papier'', **38**, 607 ff (1984) beschreibt erstmals die Synthese hochsubstituierter Trimethylsilylcellulosen im System N,N-Dimethylacetamid/Lithiumchlorid und Hexamethyldisilazan als Silylierungsmittel mit dem Ziel einer möglichst vollständigen Substitution zur Bestimmung der Molmassenverteilung mittels Gelpermeationschromatographie.

In J. Polym.Sci., Part B: Polym. Phys. **26**, 1101 ff (1988) wird die Untersuchung flüssigkristalliner Eigenschaften von Trialkylcellulosen beschrieben.

Nach "Das Papier'', **38**, 607 ff (1984) sind selbst für Substitutionsgrade von nahezu 3 nur stöchiometrische Mengen an Hexamethyldisilazan erforderlich. Bei höhermolekularen Baumwoll-Linters und Fichten-Zellstoffen stellten sich ebenfalls nur Substitutionsgrade von 2,7 ein.

In verschiedenen Literaturstellen (u.a. Z. Chem. **24**, 62 ff (1984), Z.Chem. **27**, 1 ff (1987), Makromol. Chem., Rapid Commu. **9**, 569 ff (1988), Makromol. Chem. **191**, 2985 ff (1990) und Das Papier **44**, 624 ff (1990)) werden die Nutzung und Zugänglichkeit von Trimethylsilylcellulosen als lösliche und lagerstabile Zwischenprodukte für regioselektive Homogenderivatisierungen der Cellulose beschrieben. Eine auf diese Art gesteuerte Einführung von Wirkgruppen ermöglicht es z.B., Cellulosematerialien mit definiertem Substitutionsgrad und gezielter Substituentenverteilung als Adsorbentien für die Chromatographie, Trägermatieralien für Wirkstoffe, bioaktive Polymere, flüssigkrstalline Makromoleküle, photoaktive Schichten sowie verschiedenartigste Membranen herzustellen. Hier zeigt sich das noch längst nicht ausgeschöpfte Innovationspotential auf dem Gebiet der präparativen Cellulosechemie. Dies ist, angesichts des erklärten Ziels, ausgehend von nachwachsenden Polysaccharid-Rohstoffen neue Werk- und Wirkstoffe zu schaffen, von zunehmendem Interesse.

Aufgrund ihrer guten Löslichkeit und der leichten Abspaltbarkeit der Silylether-Substituenten wurden Trimethylsilylcellulosen zu Beginn der 80er Jahre als potentielles Material für Cellulose-Regenerat-Verfahren in Betracht gezogen (vgl. Das Papier **35**, 547 ff (1981) und J. Appl. Polym. Sci **26**, 3827 ff (1981)), ohne daß es zu einer industriellen Anwendung kam. So wurde Cellulose mit Hexamethyldisilazan unter Zusatz geringer Mengen Chlortrimethylsilan/Pyridin in DMF silyliert und ein thermoplastisches Material erhalten, das sich bei 320°C aus der Schmelze zu einer Faser verspinnen ließ. Die saure Hydrolyse dieser Fasern mit verdünnten wäßrigen Säuren lieferte regenerierte Cellulose mit vergleichbaren Eigenschaften von kommerziell erhältlichen Rayon-Fasern (vgl. J. Appl. Polym. Sci **26**, 3827 ff (1981)). Da Pyridin und DMF als Reaktionsmedien ökologisch wie ökonomisch bedenklich sind und sich zudem nur schlecht aus polymeren Materialien entfernen lassen, ist versucht worden, das Xanthogenatverfahren über die löslichen Trimethylsilylcellulosen mit Ammoniak als Reaktionsmedium zu umgehen. So wurde Cellulose mit Chlortrimethylsilan in flüssigem Ammoniak umgesetzt, der sowohl als Quellmittel wie auch als Reaktionsmedium und Salzsäureakzeptor diente. Dabei wurde der Zellstoff bei Atmosphärendruck in flüssigem Ammoniak bei -70°C gequollen und mit der auf eine Trisubstitution bezogenen Menge Chlortrimethylsilan versetzt. Die Silylierung verlief aufgrund der enormen Affinität des Siliciums zum Sauerstoff in der angeführten Weise, d.h., das Chlortrimethylsilan reagierte direkt mit den Hydroxylgruppen der Cellulose. Nach beendigter Reaktion wurde der überschüssige Ammoniak zurückgewonnen und die in einigen organischen Lösungsmitteln lösliche Trimethylsilylcellulose aus dem Rückstand herausgelöst (Greber et al., Das Papier **35**, 547 ff (1981)).

Der besondere Vorteil dieses Trimethylsilylcellulose-Verfahrens besteht darin, daß damit ein Kreislauf realisierbar wird, in dem eine Rückgewinnung bzw. eine Regenerierung fast aller beteiligten Reaktanden möglich ist. Aus verfahrenstechnischer Sicht wäre selbst ein solcher funktionierender Kreislauf nicht akzeptabel, da zwar nicht mehr mit dem bedenklichen Reaktionsmedium Pyridin, aber immer noch mit dem halogenhaltigen und korrosiven Silylierungsmittel Chlortrimethylsilan gearbeitet wird. Zudem fällt bei dieser Art der Silylierung stets unlösliches Ammoniumchlorid als Nebenprodukt bzw. Verunreinigung an, welches die Isolierung und Reinigung der Trimethylsilylcellulose erschwert.

Die DE-OS 31 04 531 stellt auf ein Verfahren zur Herstellung einer neuen O-Trimethylsilyl-Cellulose eines Substitutionsgrades bis 2,0, vorzugsweise 1,4 bis 1,6 ab, wobei Holz- oder Baumwollcellulosen in trockenem, flüssigem Ammoniak gequollen und sodann mit Trimethylchlorsilan umgesetzt werden, das Reaktionsprodukt in einem stark polaren aprotischen Lösungsmittel gelöst und gegebenenfalls durch Ausfällen zu einem gereinigten trockenen Produkt verarbeitet wird. Als stark polare Lösungsmittel sollen insbesondere in Betracht kommen: Dimethylformamid (DMF), Dimethylacetamid (DMA), N-Methylpyrrolidon (NMP) und Dimethylsulfoxid (DMSO). Es wird stets, ausgenommen Beispiel 3, auf Trimethylchlorsilan als Silylierungsmittel abgestellt, wenngleich eine Ausführungsform des bekannten Verfahrens auch darin bestehen soll, "Hexamethyldisilazan vorgebildet der mit Ammoniak gequollenen Cellulose zuzusetzen". Es wird in flüssigem Ammoniak unter Normaldruck gearbeitet, der nach Abschluß der Umsetzung durch Erwärmen und Evakuieren, aber auch durch Neutralisieren entfernt werden kann. Das Reinigen soll durch Lösen in den stark polaren Lösungsmitteln, wie Dimethylacetamid und Dimethylformamid, und Abtrennen des unlöslichen Ammoniumchlorids erfolgen.

Die in der DE-OS 31 04 531 angegebenen Erfinder haben die Aussagen zum Beispiel 3 in einem später verfaßten Artikel in "Das Papier", 35, S. 551, r. Sp., 1981, widerrufen und wörtlich ausgeführt: "Die zweite Möglichkeit, daß die Silylierungsreaktion mit Hilfe von Hexamethyldisilazan erfolgt - das sich aus Trimethylchlorsilan und Ammoniak bildet (vgl. Abb. 10) - konnten wir ausschließen; denn unter verschiedenen Reaktionsbedingungen gelang die Silylierung von Cellulose in flüssigem Ammoniak mit vorgebildetem Hexamethyldisilazan nicht'' (a.a.O., S. 551, r. Sp., Abs. 2). Hiermit haben sie mit Nachdruck die Eignung von Hexamethyldisilazan zur Silylierung von Kohlenhydraten in flüssigem Ammoniak bei Normaldruck bestritten. Sie lassen also keinen Zweifel daran, daß die in Beispiel 3 der DE-OS 31 04 531 wiedergegebene technische Lehre nicht ausführbar ist.

Die US- PS 4 390 692 beschreibt die Herstellung von Trimethylsilylcelluloseestern unter Einsatz von Hexamethyldisilazan. Es wird darin darauf hingewiesen, daß die meisten vorbekannten Verfahren eine große Menge an Lösungsmitteln benötigen, um die Cellulose zu dispergieren, die Lösungsmittel danach von dem silylierten Celluloseprodukt abgetrennt werden müssen. Daher soll die Lösungsmittelmenge herabgesetzt werden und ein hoher Silylierungsgrad angestrebt werden, der allerdings tatsächlich höchstens 2,19 zu betragen scheint. Um die angesprochenen Probleme zu lösen, wird die Silylierung mit Hexamethyldisilazan in Gegenwart einer kleinen Menge eines Katalysators durchgeführt. Als "Katalysatoren" werden u.a. Acetamid und N,N-Dimethylformamid, stark polare aprotische Lösungsmittel, eingesetzt. Vorteilhaft soll es sein, neben diesen stark polaren Lösungsmitteln ein (als korrosiv bekanntes) Ammoniumhalogenid, beispielsweise Ammoniumchlorid, einzusetzen. Die Lehre nach der US-PS 4 390 692 setzt also stark polare Lösungsmittel in einer verhältnismäßig kleinen Menge ein, um ersichtlich zumindest eine Benetzungsphase für die Silylierungsreaktion zu bilden. Daß es sich hierbei nicht um "katalytische" Mengen im üblichen Sinne handeln kann, ergibt sich unmittelbar aus dem in der US-PS 4 390 692 angegebenen Gewichtsverhältnis von Hexamethyldisilazan zu beispielsweise N,N-Dimethylformamid von etwa 10:1 bis 20:1. Um die Lösungs- und nicht die "Katalysator"-funktion der stark polaren Lösungsmittel zu verbessern, sollen die Reaktionstemperaturen auf etwa 100 bis 135°C eingesetellt werden.

Auch die Literaturstelle "Journal of Polymer Science" Part A-1, Vol. 7, (1969), 1947-1958 hebt beim Einsatz von Hexamethyldisilazan als Silylierungsmittel von Kohlenhydraten ausdrücklich auf das Arbeiten in einem hochsiedenden stark polaren Lösungsmittel ab. Diese Lösungsmittel sind schwer entfernbar und regelmäßig toxisch.

Der Erfindung lag die Aufgabe zugrunde, ein Verfahren zur Silylierung von Kohlenhydraten in flüssigen Stickstoffverbindungen zu schaffen, das unter ökonomischen wie auch ökologischen Gesichtspunkten Vorteile bietet, die Herstellung eines Verfahrenserzeugnisses hohen Substitutions- bzw. Silylierungsgrades sowie verbesserter Reinheit ermöglicht, das in vorteilhafter Weise schmelzversponnen werden kann, wobei die erhaltenen Fasern sowie die daraus erhältlichen desilylierten Fasern gegenüber Vergleichsprodukten verbesserte Reißfestigkeitswerte aufweisen. Insbesondere soll auf den Einsatz schwer entfernbarer, stark polarer Lösungsmittel verzichtet werden können.

Erfindungsgemäß wird diese Aufgabe dadurch gelöst, daß 10 Gew.-Teile Kohlenhydrate unter Druck in einem Autoklaven bei einer Temperatur von 25 bis 150°C mit einem Silylierungsmittel der Formel (I) 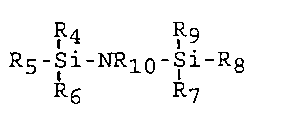 in mindestens 1 Gew.-Teil einer flüssigen Stickstoffverbindung der Formel (II) 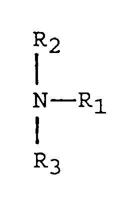 umgesetzt werden, wobei in den beiden Formeln (I) und (II) die jeweiligen Reste, unabhängig voneinander, sind:
Wasserstoff, eine niedere Alkylgruppe mit 1 bis 4 Kohlenstoffatomen, eine Cycloalkylgruppe mit 3 bis 6 Kohlenstoffatomen, eine Arylgruppe in Form eines Phenyl- oder Naphthyl-Rests; eine Aralkylgruppe mit 7 bis 18 Kohlenstoffatomen, eine Alkylarylgruppe mit 7 bis 18 Kohlenstoffatomen oder eine O-, S- oder N-haltige heterocyclische Gruppe mit 2 bis 5 Kohlenstoffatomen.

Vorzugsweise wird die Umsetzung zwischen den beiden Verbindungen der Formel (I) und (II) in einem Temperaturbereich von 50 bis 120°C durchgeführt. Dabei ist es wichtig, daß die Umsetzung in einem Autoklaven bzw. einem Gefäß zum Erhitzen unter Druck erfolgt, so daß bei erhöhter Temperatur zumindest ein ausreichender Teil der Stickstoffverbindung der Formel (II) in flüssigem Zustand vorliegt. Dabei sollte die Menge der flüssigen Phase dieser Stickstoffverbindung ausreichen, um das zu silylierende Kohlenhydrat zumindest so weit zu benetzen, so daß in der Benetzungsphase die gewünschte Reaktion abläuft. Es entfallen auf 10 Gew.-Teile zu silylierendes Kohlenhydrat, insbesondere in Form von Cellulose, mindestens 1 Gew.-Teil Ammoniakverbindung, vorzugsweise mindestens 3 Gew.-Teile und insbesondere 5 Gew.-Teile flüssige Stickstoffverbindung, insbesondere in Form von Ammoniak. Aus wirtschaftlichen Gründen sollte diese Mengenrelation optimiert werden, da ein Überschuß an flüssiger Stickstoffverbindung keine Vorteile bietet. Vorzugsweise läuft die Umsetzung in einer kontinuierlichen Phase der flüssigen Stickstoffverbindung ab, da die flüssige Stickstoffverbindung für die nicht löslichen Kohlenhydrate nicht nur als Reaktionsmedium, sondern auch als Quellmittel dient. Sie greift darüber hinaus auch noch in die Reaktionsabläufe ein, da sie ein Protonenakzeptor ist und damit, wie zu vermuten ist, zumindest die Ablösung des Protons der Hydroxylgruppe des Kohlenhydrats begünstigt, so daß der Sauerstoff dieser Gruppe dann aufgrund seiner starken Affinität zu dem Silicium-Atom die angestrebte Silylierung optimiert.

Um eine Optimierung des erfindungsgemäßen Verfahrens zu erreichen, wird es bevorzugt, das Silylierungsmittel sowie die flüssige Stickstoffverbindung, die jeweils gewählt werden, aufeinander abzustimmen. Vorzugsweise sind zwei der Reste R₁, R₂ und R₃ Wasserstoff, und ein Rest hiervon ist identisch mit dem später noch erläuterten Rest R₁₀ des Silylierungsmittels der Formel (I). Dies führt dazu, daß mit Abschluß des Silylierungsvorganges die Gruppe "NR₁₀" in substituiertes Ammoniak der Formel NH₂R₁₀ überführt wird und dann R₁₀ = beispielsweise R₁ der Formel (II) bei R₂ und R₃ = Wasserstoff ist. Hierbei wird bei Abschluß der Silylierungsreaktion aus dem geöffneten Autoklaven eine einheitliche flüssige Stickstoffverbindung abgeführt. Von besonderem Vorteil ist es, wenn als flüssige Stickstoffverbindung der Formel (II) das unter den Verfahrensbedingungen flüssige Ammoniak verwendet wird, so daß R₁₀ dann in der Formel (I) des Silyierungsmittels ebenfalls Wasserstoff ist.

In den obigen Formeln (I) und (II) sind die jeweiligen Reste vorzugsweise Wasserstoff, da die entsprechenden Ausgangsverbindungen handelsüblich bzw. leichter herstellbar sind. Geeignet sind jedoch im Rahmen der Erfindung auch die weiteren Möglichkeiten, wie sie bei der vorstehenden Erfindungsdefinition wiedergegeben sind. Als eine niedere Alkylgruppen mit 1 bis 4 Kohlenstoffatomen kommen die Methyl-, Ethyl-, n-Propyl-, i-Propyl- sowie die verschiedenen Isomeren der Butylgruppe in Frage. Unter die Cycloalkylgruppe mit 3 bis 6 Kohlenstoffatomen fallen im Rahmen der Erfindung insbesondere der Cyclopropyl-, Cyclobutyl sowie Cyclopentylrest, unter die Aralkylgruppe mit 7 bis 18 Kohlenstoffatomen insbesondere die Benzyl- und Phenethylgruppe, unter die Alkylarylgruppe mit 7 bis 18 Kohlenstoffatomen insbesondere die Tolylgruppe sowie unter die heterocyclische Gruppe mit 2 bis 5 Kohlenstoffatomen insbesondere solche, in deren heterocyclischem Ring sich mindestens ein Sauerstoff-, Schwefel- oder ein N-Atom befindet, wobei als geeignete Beispiele die Radikale des Oxirans, Tetrahydrofurans, Dioxans sowie Pyrans angegeben werden können. Entscheidend ist es für die oben angesprochenen Substituenten, daß sie die im Rahmen der beim erfindungsgemäßen Verfahablaufenden chemischen Mechanismen gewährleisten bzw. nicht stören. Sie sollen demzufolge im Sinne der Erfindung die Silylierung der Kohlenhydrate ohne weiteres ermöglichen und darüber hinaus auch die möglichen Weiterverarbeitungen der silylierten Kohlenhydrate, beispielsweise die Desilylierung oder Umsetzung mit weiteren Reagenzien ermöglichen. Dabei sollen die obigen Definitionen der verschiedenen Reste R₁ bis R₁₀ auch die Möglichkeit einbeziehen, daß ebenfalls nicht störende Substituenten eingeführt werden, hier beispielsweise wiederum eine niedere Alkylgruppe mit 1 bis 4 Kohlenstoffatomen oder geeignete Halogenatome, wie Chlor.

Vorzugsweise ist die unter den Verfahrensbedingungen flüssige Stickstoffverbindung der Formel (II) Ammoniak oder ein primäres, sekundäres oder tertiäres Amin, wie insbesondere Methyl- bzw. Ethylamin, Dimethyl- bzw. Diethylamin und Trimethyl- bzw. Triethylamin.

Die obigen Ausführungen zu den Substituenten R₁ bis R₃ bezüglich der in Frage kommenden Substituenten gelten entsprechend für die Reste R₄ bis R₁₀ der Formel (II) des Silylierungsmittels. Ein ganz besonders geeignetes Silylierungsmittel ist das Hexamethyldisilazan, das zu vorzüglichen Silylierungen führt, und vorzugsweise in flüssigen Ammoniak eingesetzt wird.

Es ist überraschend, daß trotz der klaren Aussagen in "Das Papier", **35**, S. 551 r.Sp. (1981), erfindungsgemäß in höchst vorteilhafter Weise die Silylierung von Kohlenhydraten mit Hexamethyldisilazan in Ammoniak gelingt, wobei dies ohne den Einsatz stark polarer Lösungsmittel möglich ist und ein Verfahrenserzeugnis mit einem Silylierungsgrad bis zu 3 erhältlich ist. Einzelheiten über die nicht erfolgreich durchgeführten Verfahren finden sich in dieser Literaturstelle nicht.

Das quantitative Verhältnis von Silylierungsmittel zu dem jeweils gewählten Kohlenhydrat ist für die Durchführung des erfindungsgemäßen Verfahrens nicht kritisch. Das Mengenverhältnis richtet sich nach dem angestrebten oder auch höchstmöglichen Substitutionsgrad. Dem Fachmann ist es ohne weiteres möglich, das jeweilige optimale Verhältnis von Silylgruppen des eingesetzten Silylierungsmittels zu den OH-Gruppen der Monomer-Einheiten des jeweiligen Kohlenhydrats, beispielsweise der Anhydroglucose-Einheiten in Stärke oder Cellulose, anhand einfacher Versuche festzulegen. Dabei spielt im Einzelfall auch das Cellulose- bzw. Stärkeausgangsmaterial eine Rolle. So könnte man für die erfindungsgemäß eingesetzten Silylierungsmittel, insbesondere in Form von Hexamethyldisilazan, das Verhältnis von Silylgruppen aus eingesetztem Silylierungsmittel pro OH-Gruppe der Anhydroglucose-Einheiten des jeweiligen Polysaccharids, insbesondere von Cellulose, mit vorzugsweise 5:1 bis 1:1, insbesondere 3:1 bis 1:1, ansetzen, um, wie die nachfolgende Tabelle I zeigt, einen Substitutionsgrad von mindestens 1,9 oder 2,1 und insbesondere von 2,5 oder mehr zu erzielen. Eine Anhebung der Menge an Silylierungsmittel, insbesondere an Hexamethyldisilazan über das angesprochene Verhältnis von 5:1 führt zu keiner Steigerung des Substitutionsgrades. Bei einer optimalen Verfahrensführung sollte die Menge an jeweiligem Silylierungsmittel so gewählt werden, daß es möglichst vollständig aufgebraucht wird, so daß nach Ableiten des Reaktionsmediums, insbesondere in Form von Ammoniak, lediglich das Reaktionsprodukt in dem Autoklaven verbleibt.

In Einzelfällen kann es bei der Durchführung des erfindungsgemäßen Verfahrens von Vorteil sein, daß in dem Reaktionsmedium nicht nur das Silylierungsmittel, das zu silylierende Kohlenhydrat sowie die unter den Reaktionsbedingungen flüssige Stickstoffverbindung vorliegen, sondern in das Reaktionsmedium zusätzlich inerte Lösungsmittel hinzugegeben werden, wie Hexan, Toluol und THF (Tetrahydrofuran). Es handelt sich um inerte Lösungsmittel, die bei Raumtemperatur von etwa 20°C flüssig sind. Nach Abdampfen der flüssigen Stickstoffverbindung, insbesondere in Form von Ammoniak, bleiben diese inerten Lösungsmittel zurück und überführen die silylierten Kohlenhydrate in eine Lösung, die weiterverarbeitet werden kann. Beispielsweise kann zu diesen Lösungen ein polares Lösungsmittel, wie Methanol oder Ethanol gegeben werden, so daß das silylierte Erzeugnis auf diese Weise ausgefällt wird. So kann es in einer geeigneten Lösung naß oder trocken versponnen werden. Dies gilt zwar auch für die vorstehend abgehandelten und nach bekannten Verfahren erhaltenen Erzeugnisse, die jedoch nicht gleichermaßen rein anfallen.

Die obigen Ausführungen zeigen, daß die erhaltenen Verfahrenserzeugnisse in polaren Lösungsmitteln unlöslich, jedoch in unpolaren Lösungsmitteln löslich sind. Dies ist ein besonderes Charakteristikum der erfindungsgemäß erhaltenen silylierten Kohlenhydrate, insbesondere in Form silylierter Cellulose. Unter "unpolaren Lösungsmitteln" im Sinne der Erfindung sollen insbesondere solche verstanden werden, bei denen die Dielektrizitätszahl maximal 3 beträgt, wozu bezüglich der Festlegung der Dielektrizitätszahl auf Römpp Chemie Lexikon, 9. Aufl., Bd. 2, 1990, S. 955, r.Sp. - S. 956, 1. Sp., verwiesen sei. Hierunter fallen beispielsweise Benzol und Tetrachlorkohlenstoff, deren Dielektrizitätszahl 2,3 bzw. 2,2 beträgt. Demgegenüber haben die als polar bzw. gar stark polar zu bewertenden Lösungsmittel Methanol, Nitrobenzol und Formamid Dielektrizitätszahlen von 33,6, 35,7 sowie 109.

Das erfindungsgemäß erhaltene reine Erzeugnis läßt sich beliebigen weiteren Umsetzungen unterziehen. So können die verbliebenen freien Hydroxylgruppen beispielsweise mit Acylierungsmitteln umgesetzt werden, wobei insbesondere Acetylierungsmittel in Frage kommen. Zu den geeigneten Acetylierungsmitteln zählen insbesondere Essigsäureanhydrid und Acetylchlorid, wobei die Acetylierung in üblicher Weise und vorzugsweise in Gegenwart eines Lösungsmittels, wie z.B. Benzol, Essigsäure u. dgl., gegebenenfalls auch in Gegenwart von Katalysatoren erfolgt. Die Acetylierung ist nicht Gegenstand der vorliegenden Erfindung. Es soll allein gezeigt werden, daß sich die erfindungsgemäß erhältlichen reinen Erzeugnisse hierzu besonders eignen.

Das erfindungsgemäße Verfahren ist im Hinblick auf die Art der zu silylierenden Kohlenhydrate nicht kritisch eingeschränkt. Grundsätzlich können danach Mono-, Di- und Polysaccharide silyliert werden. Besonders geeignet ist das erfindungsgemäße Verfahren zur Silyierung von Saccharose, Stärke und Cellulose sowie von davon abgeleiteten Produkten, die mehr oder weniger weit abgebaut sind, beispielsweise Dextrine.

An die Art des bei der Durchführung des erfindungsgemäßen Verfahrens eingesetzten Autoklavs sind keine besonderen Anforderungen zu stellen. Der Begriff "Autoklav" soll im Sinne der Erfindung weitestgehend verstanden werden. Entscheidend ist es, daß in der jeweils gewählten Reaktionsapparatur während der Reaktion der erforderliche Druck eingestellt wird, um die für das erfindungsgemäße Verfahren wichtigen Verfahrensparameter einhalten zu können. Somit könnte man unter einem Autoklaven im Sinne der Erfindung auch einen geeignet konzipierten Extruder verstehen, bei dem beispielsweise Ammoniak unter Druck verflüssigt ist und auch die geeigneten Temperaturen vorliegen. Dies ermöglicht auch eine vorteilhafte kontinuierliche Verfahrensführung. Vorzugsweise wird ein Autoklav eingesetzt, der zur guten Durchmischung der Reaktionspartner eine geeignete Rührvorrichtung, um damit insbesondere die Reaktionszeiten zu verkürzen, aufweist.

Vorstehend wurde bereits gezeigt, daß es für die Durchführung des erfindungsgemäßen Verfahrens zur Silylierung von Kohlenhydraten bevorzugt ist, auf Feuchtigkeitsausschluß zu achten, was insbesondere für niedermolekulare Kohlenhydrate zutrifft. Dies gilt auch für die Handhabung der verschiedenen Reaktionsprodukte aufgrund ihrer großen Hydrolyseempfindlichkeit. Vorzugsweise wird der Feuchtigkeitsausschluß durch vorausgegangenes ausreichendes Trocknen der Reaktionspartner erreicht. So wurden Versuche mit feuchtigkeitsempfindlichen Substanzen in ausgeheizten und unter Schutzgas abgekühlten Apparaturen durchgeführt. Als Schutzgas diente Argon (Schweiß-Argon 4.6), das über eine Normag-Gasreinigungsanlage gereinigt und getrocknet wurde. Glasgeräte der Firma Normag wurden auch für die präparativen Arbeiten herangezogen. Die Reaktionen in flüssigem Ammoniak (99,8%) erfolgten in einem Edelstahlautoklaven (Werkstoffnummer 1.4571) mit einem Innenvolumen von 55 cm³. Ammoniak wurde in flüssiger Form eingepreßt. Alle hygroskopischen und mit Wasser mischbaren Lösungsmittel wurden vor Verwendung IR-spektroskopisch auf Abwesenheit von Feuchtigkeit geprüft. Sämtliche Kohlenhydrate, wie Cellulose (Baumwoll-Linters und Fichten-Zellstoff der Firma Fluka), verschiedene Stärken und Dextrine (Firma Cerestar, Firma Roquette) sowie Saccharose, wurden im Ölpumpenvakuum bei 70°C bis zur Gewichtskonstanz getrocknet. Alle übrigen Chemikalien wurden, falls bei den nachfolgenden Beispielen in der Versuchsbeschreibung nichts anderes angegeben, ohne weitere Reinigung eingesetzt. Das erfindungsgemäße Verfahren kann auch bei Vorliegen einer geringen Feuchtigkeitsmenge erfolgreich durchgeführt werden, da kleine Wassermengen ohne weiteres durch ein im Überschuß vorhandenes Silylierungsmittel verbraucht werden.

Das erfindungsgemäße Verfahren vermeidet die mit den bekannten Verfahren im Zusammenhang mit Chlortrimethylsilan verbundenen Korrosionsprobleme. Darüber hinaus findet kein Abbau bei der Silylierung statt. Der Polymerisationsgrad bleibt demzufolge konstant.

Die nach dem erfindungsgemäßen Verfahren erhaltenen Erzeugnisse sind bezüglich ihrer chemischen Eigenarten mit den eingangs geschilderten bekannten Erzeugnissen chemisch-strukturell vergleichbar. Sie weisen jedoch unterscheidende Besonderheiten auf, so insbesondere die Unlöslichkeit in polaren Lösungsmitteln. Insbesondere sind sie weitgehend frei von Verunreinigungen, was für die bekannten Verfahrenserzeugnisse nicht gilt.

Die Reinheit ergibt sich daraus, daß zur Herstellung weniger Substanzen erforderlich sind und die eingesetzte Verbindungsgruppe der angesprochenen Formel (II), insbesondere in Form von Ammoniak, problemlos abgedampft werden kann. Diese Reinheit bietet Vorteile bei Weiterverarbeitungen, so beispielsweise bei der gängigen Herstellung von Regenerat-Cellulosefasern. Dabei lassen sich die Regenerat-Cellulosefasern sowohl nach dem Schmelz- als auch nach dem Naßspinnverfahren gewinnen. Bei dem Schmelzspinnverfahren wird die silylierte Cellulose in die Schmelze überführt und zu einem dünnen Faden versponnen. Anschließend wird dieser dünne Faden in einem sauren Medium desilyliert. Beim Naßspinnverfahren wird die silylierte Cellulose in einem geeigneten Lösungsmittel, insbesondere in Form von Hexan, Toluol und Tetrahydrofuran, gelöst und der Lösungsstrahl in ein saures Fällbad geleitet, insbesondere in Form eines Gemisches aus Isopropanol, Wasser und HCl oder Methanol, Wasser und HCl. Diese Maßnahmen sind, wie es aus der Beschreibungseinleitung hervorgeht, Stand der Technik. Es hat sich jedoch gezeigt, daß die auf diese Weise aus den erfindungsgemäß silylierten Ausgangsmaterialien erhaltenen Regenerat-Cellulosefasern günstige Reißfestigkeitswerte aufweisen. Das angesprochene Xanthogenat-Verfahren steht wegen seiner starken Umweltbelastung unter erheblichem Druck. Die Abwässer enthalten Natriumsulfat, Zinksulfat, Schwefelsäure und nicht unbedeutende Mengen an Schwefelkohlenstoff und Schwefelwasserstoff, die unter großem technischen und finanziellen Aufwand vor dem Einleiten in die Vorfluter entfernt werden müssen.

Ein weiterer Vorteil des erfindungsgemäßen Verfahrens liegt darin, daß Ammoniak im Falle seines Einsatzes durch Drucksenkung bzw. Öffnung des Autoklavs nicht nur leicht abgedampft, sondern später ohne weiteres dem Verfahrenszweck wieder zugeführt werden kann. Das gleiche gilt für die eingesetzten Silylierungsmittel, insbesondere für das Hexamethyldisilazan, das nach bekannten Techniken aus dem entstandenen Hexamethyldisiloxan zurückgewonnen werden kann. Hierzu sei auf Journal of Applied Polymer Science Bd. 26, 3832 (1981), John Wiley & Sons, Inc., verwiesen. Schließlich läßt sich erfindungsgemäß ein höherer Silylierungsgrad erreichen, insbesondere von mehr als 2,5 und, wenn gewünscht, bis zu 3. Dies zeigen die nachfolgend wiedergegebenen Beispiele bzw. Vergleichsbeispiele. Dabei wurde, um einen objektiv zutreffenden Vergleich erzielen zu können, stets von gleichen Celluloseausgangsmaterialien ausgegangen. Ein höherer Substitutionsgrad führt zu einer besseren Wärmebeständigkeit. Dies er möglicht ein höheres Aufheizen mit der Folge einer günstigen Viskosität beim Verspinnen in der Schmelze.

Die vorliegende Erfindung soll anhand der nachfolgenden Beispiele noch näher erläutert werden.

### Beispiel 1 (Herstellung von Octakis-(trimethylsilyl)-saccharose)

In dem ausgeheizten Autoklaven mit Magnetrührstab werden unter Feuchtigkeitsausschluß 3,61 g (10,55 mmol) Saccharose und 0,077 g (0,42 mmol) Saccharin (0,5 mol% bzgl. der OH-Gruppen der Saccharose) mit 10,7 ml (50,61 mmol) Hexamethyldisilazan versetzt. Nach Einpressen von 27,5 g Ammoniak wird der Autoklav auf 50°C temperiert und die Reaktionsmischung 14 Stunden lang gerührt. Wegen der großen Hydrolyseempfindlichkeit des Reaktionsproduktes ist bei allen Arbeitsoperationen streng auf Feuchtigkeitsausschluß zu achten. Man läßt auf Raumtemperatur abkühlen und dampft den Ammoniak durch vorsichtiges Öffnen des Einleitungsventiles langsam ab. Nach dem Abdampfen des Ammoniaks wird das zähflüssige braune Rohprodukt in 100 ml n-Hexan gelöst. Man dekantiert von festen Verunreinigungen ab und entfernt durch Destillation bei Normaldruck das n-Hexan. Das zurückbleibende gelbe Öl unterwirft man einer Hochvakuumdestillation in einer Kugelrohrdestillationsapparatur. Die Ausbeute an Produkt betrug 7,7 g (79% d. Theorie). Die Eigenschaften des Verfahrensproduktes können wie folgt beschrieben werden:
Kp_{0,05}: 200°C (nach J. org. Chem. **23**, 773 (1958)): 190-200°C
n²⁰_{D}: 1.4440 (nach J. org. Chem. **23**, 773 (1958) und Makromol. Chem. **24**, 1 (1957): 1.4434)
IR(_{Film}): 1256, 845 und 750 cm⁻¹ (Si-CH₃)
¹H-NMR: 0,10-0,18 (6s, 72H), 3,25-3,96 (m, 12H)
(CDCl₃): 4,28 (d, 1H), 5,21 ppm (d, 1H)

### Beispiel 2 (Silylierung von Polysacchariden)

Es werden entsprechend der nachfolgenden Tabelle I verschiedene Polysaccharide, Hexamethyldisilazan und Ammoniak in den Autoklaven eingewogen und bei unterschiedlichen Reaktionsbedingungen umgesetzt. Diese Umsetzung erfolgte im Rahmen der Angaben des vorstehenden Beispiels 1 unter Beachtung der sich aus der angesprochenen Tabelle I ergebenen Reaktionsbedingungen. Man läßt auf Raumtemperatur abkühlen und dampft den Ammoniak durch vorsichtiges Öffnen des Einleitungsventils langsam ab. Anschließend wird das im Autoklaven zurückbleibende Reaktionsprodukt im Ölpumpenvakuum bei 60°C von flüchtigen Bestandteilen befreit. Dieses Rohprodukt wird in unpolaren Lösungsmitteln, wie n-Hexan, Cyclohexan oder Toluol, gelöst. Dabei bilden sich extrem viskose Lösungen, die bei Konzentrationen größer als 0,5 Gew.-% feinverteilte, lediglich gequollene Gelanteile enthalten, die sich über Nacht am Boden des Geraßes absetzen. Bei Raumtemperatur gelieren diese Lösungen ab ca. 5 Gew.-%. Nach dem Ausfällen mit Ethanol wird der weiße Feststoff abgesaugt, mit Ethanol gewaschen und im Ölpumpenvakuum bei 70°C getrocknet.

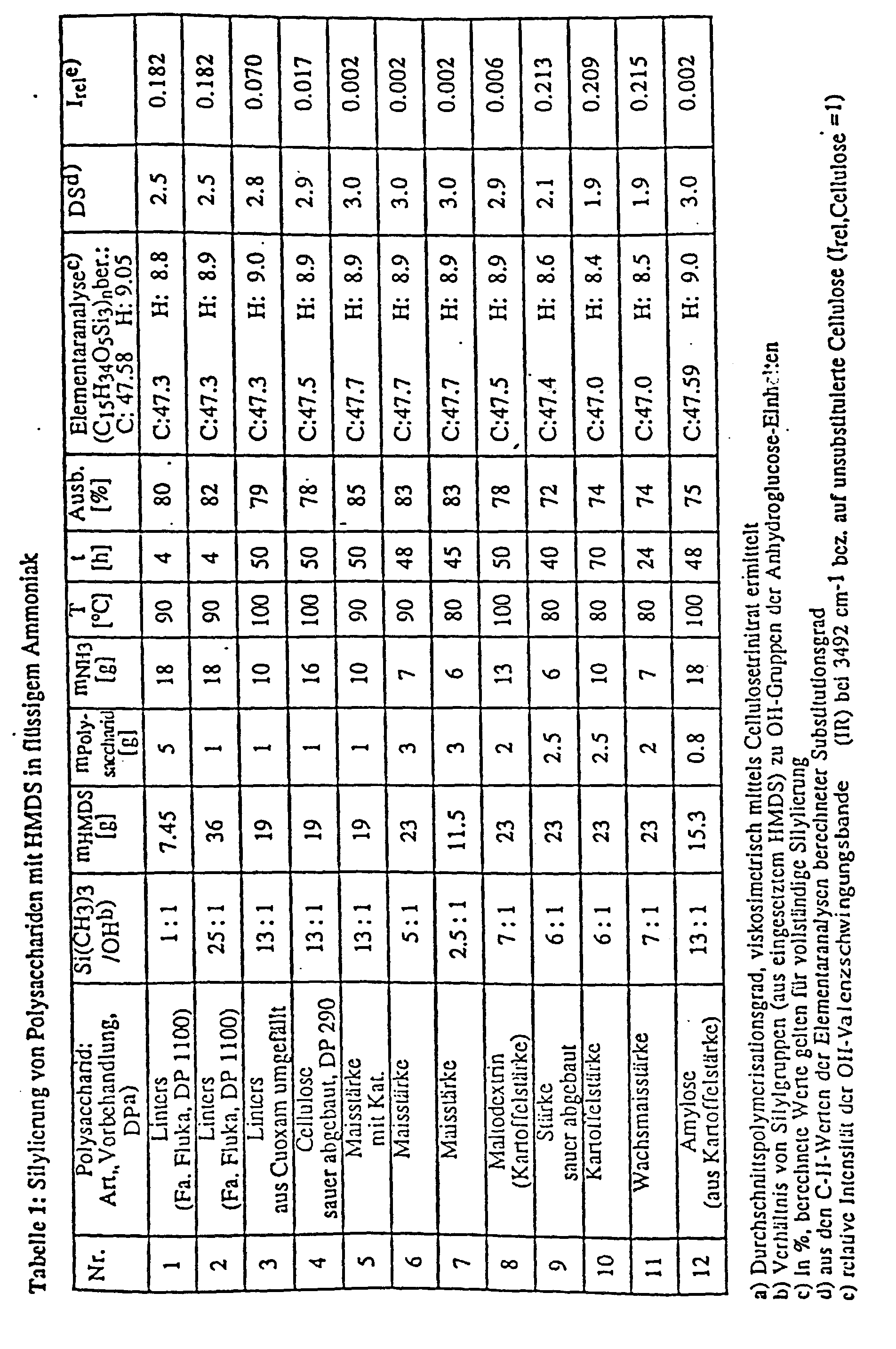

### Vergleichsbeispiel 1 (Silylierung von Cellulose (Baumwoll-Linters) / Vergleich zum Beispiel 2)

### Methode a (beschrieben u.a. in J.Am.Chem. Soc. 70, 1919 ff (1948))

In einem unter Argon ausgeheizten 250 ml Zweihalsschutzgaskolben mit Magnetrührstab, Tropftrichter, Rückflußkühler und Calciumchlorid-Trockenrohr werden 2 g (12,3 mmol) Cellulose in 60 ml trockenem Pyridin suspendiert und 1 Stunde am Rückfluß erhitzt. Man kühlt auf Raumtemperatur ab und tropft innerhalb 30 min 8 g (73,6 mmol) Chlortrimethylsilan, gelöst in 50 ml n-Hexan, zu. Nach 8 Stunden Rühren bei Raumtemperatur erhält man eine leicht trübe viskose Lösung, die in 300 ml Methanol eingegossen wird. Der dabei ausfallende weiße faserartige Feststoff wird abgesaugt, zweimal mit je 100 ml Methanol gewaschen und 6 Stunden lang im Ölpumpenvakuum (0,06 mbar) bei 65°C getrocknet. Zur weiteren Reinigung wird aus n-Hexan in Ethanol gefällt und erneut getrocknet. Die Ausbeute beträgt 3,96 g (85% der Theorie). Die Eigenschaften des Produktes waren wie folgt:
IR_{(Film)}: 3492 cm⁻¹ (O-H), 1255, 840 und 755 cm⁻¹ (Si-CH₃)
Elementar-analyse: ber. C: 47,58 H: 9,05 (C₁₅H₃₄O₅Si₃)ₙ M=378,65 g/mol
gef. C: 47,2 H: 8,4
Substitutionsgrad: 2,3

### Methode b (beschrieben u.a. in Carbohyd. Res. 31, 407 ff (1973))

In einem unter Argon ausgeheizten 500 ml Einhalsschutzgaskolben mit Magnetrührstab, aufgesetztem Tropftrichter, Blasenzähler und Calciumchlorid-Trockenrohr werden 5 g (30,8 mmol) Cellulose in 150 ml trockenem Formamid 6 Stunden bei 80°C gerührt. Die Lösung wird auf Raumtemperatur abgekühlt und unter Rühren langsam mit 100 ml (0,47 mol) Hexamethyldisilazan versetzt. Nach beendeter Zugabe wird die Temperatur für 2 Stunden auf 70°C gesteigert. Die viskose Mischung wird auf Raumtemperatur abgekühlt und unter intensivem Rühren in 500 ml wasserfreies Aceton gegossen. Das weiß ausfallende Produkt wird abfiltriert, mehrfach mit Aceton gewaschen und im Ölpumpenvakuum 3 Stunden bei 50°C getrocknet. Die Ausbeute betrug 10,19 g (87% der Theorie). Die Eigenschaften waren wie folgt:
IR_{(Film)}: 3492 cm⁻¹ (O-H), 1255, 840 und 755 cm⁻¹ (Si-CH₃)
Elementar-analyse: ber. C: 47,58 H: 9,05 (C₁₅H₃₄O₅Si₃)n M=378,65 g/mol
gef. C: 46,9 H: 8,2
Substitutionsgrad: 1,9

### Methode c (beschrieben in Polym.Sci.Part A-1 7, 1947 ff (1969))

In einen unter Argon ausgeheizten 500 ml Zweihalsschutzgaskolben mit Magnetrührstab, Tropftrichter, Rückflußkühler und Calciumchlorid-Trockenrohr werden 1,2 g (7,4 mmol) Cellulose in einem Gemisch von 100 ml trockenem Pyridin und 100 ml trockenem Dimethylformamid mit 10 ml (59 mmol) N,O-Bis(trimethylsilyl)acetamid bei einer Ölbadtemperatur von 150-160°C zur Reaktion gebracht. Nach 1 Stunde gibt man 150 ml Toluol hinzu und rührt die Mischung weitere 4 Stunden bei 150°C. Die trübe Mischung wird auf Raumtemperatur abgekühlt und unter starkem Rühren in 1,5 l Methanol gegeben. Die ausfallenden weißen Fasern werden mit Methanol gewaschen und im Ölpumpenvakuum bei 50°C getrocknet. Die Ausbeute betrug 1,61 g (70% der Theorie). Die Eigenschaften sind wie folgt:
IR_{(Film)}: 3492 cm⁻¹ (O-H), 1255, 840 und 755 cm⁻¹ (Si-CH₃)
Elementar-analyse: ber. C: 47,58 H: 9,05 (C₁₅H₃₄O₅Si₃)ₙ M=378,65 g/mol
gef. C: 47,0 H: 8,5
Substitutionsgrad: 2,0

### Methode d (beschrieben in "Das Papier" 38, 607 ff (1984))

In einem 100 ml Einhalskolben wird 1 g Cellulose in 50 ml Wasser aufgeschlämmt und über Nacht im Kühlschrank aufbewahrt. Die Cellulose wird scharf abgesaugt, in 50 ml Dimethylacetamid suspendiert und nach 30 min erneut durch Filtration isoliert. Man resuspendiert in 50 ml Dimethylacetamid und läßt die erhaltene Suspension wird über Nacht im Kühlschrank stehen. Die so aktivierte Cellulose wird abfiltriert und lösungsmittelfeucht eingesetzt.

In einem unter Argon ausgeheizten 250 ml Zweihalsschutzgaskolben mit Magnetrührstab, Tropftrichter, Rückflußkühler und Calciumchlorid-Trockenrohr wird 1 g (6,2 mmol) der aktivierten Cellulose in einer Lösung von 5 g Lithiumchlorid in 100 ml trockenem Dimethylacetamid gelöst. Die Lösung wir unter Rühren auf 80°C erwärmt und bei dieser Temperatur innerhalb 1 Stunde mit 10 ml (47 mmol) Hexamethyldisilazan versetzt. Der dabei ausfallende farblose Niederschlag wird nach dem Abkühlen der Reaktionsmischung auf Raumtemperatur abgesaugt, mehrfach mit Methanol gewaschen und im Ölpumpenvakuum bei 50°C getrocknet.

Die Ausbeute beträgt 1,61 g (70% der Theorie). Die Eigenschaften des Produktes sind wie folgt:
IR_{(Film)}: 3492 cm⁻¹ (O-H), 1255, 840 und 755 cm⁻¹ (Si-CH₃)
Elementaranalyse: ber. C: 47,58 H: 9,05 (C₁₅H₃₄O₅S1₃)ₙ M=378,65 g/mol
gef. C: 46,9 H: 8,5
Substitutionsgrad: 2,0

## Patentansprüche

1. Verfahren zur Silylierung von Kohlenhydraten in flüssigen Stickstoffverbindungen, dadurch gekennzeichnet, daß 10 Gew.-Teile Kohlenhydrate unter Druck in einem Autoklaven bei einer Temperatur von 25 bis 150°C mit einem Silylierungsmittel der Formel (I) 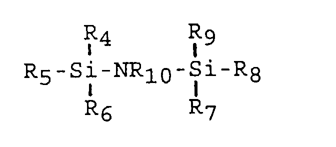 in mindestens 1 Gew.-Teil einer flüssigen Stickstoffverbindung der Formel (II) 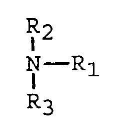 umgesetzt werden, wobei in den beiden Formeln (I) und (II) die jeweiligen Reste, unabhängig voneinander, sind:
Wasserstoff, eine niedere Alkylgruppe mit 1 bis 4 Kohlenstoffatomen, eine Cycloalkylgruppe mit 3 bis 6 Kohlenstoffatomen, eine Arylgruppe in Form eines Phenyl- oder Naphthyl-Restes, eine Aralkylgruppe mit 7 bis 18 Kohlenstoffatomen, eine Alkylarylgruppe mit 7 bis 18 Kohlenstoffatomen oder eine O-, S- oder N-haltige heterocyclische Gruppe mit 2 bis 5 Kohlenstoffatomen.

2. Verfahren nach Anspruch 1, dadurch gekennzeichnet, daß die Umsetzung bei einer Temperatur von 50 bis 120°C durchgeführt wird.

3. Verfahren nach Anspruch 1 oder 2, dadurch gekennzeichnet, daß die Umsetzung unter weitgehendem Feuchtigkeitsausschluß durchgeführt wird.

4. Verfahren nach Anspruch 3, dadurch gekennzeichnet, daß der Feuchtigkeitsausschluß durch vorausgegangenes ausreichendes Trocknen der Reaktionspartner erreicht wird.

5. Verfahren nach mindestens einem der vorhergehenden Ansprüche, dadurch gekennzeichnet, daß in der Formel (II) der Stickstoffverbindung die Reste R₁, R₂ oder R₃ eine Methylgruppe und/oder Wasserstoff sind.

6. Verfahren nach Anspruch 5, dadurch gekennzeichnet, daß als Stickstoffverbindung der Formel (II) Ammoniak eingesetzt wird.

7. Verfahren nach mindestens einem der vorhergehenden Ansprüche, dadurch gekennzeichnet, daß als Silylierungsmittel Hexamethyldisilazan eingesetzt wird.

8. Verfahren nach mindestens einem der vorhergehenden Ansprüche, dadurch gekennzeichnet, daß in dem Reaktionsmedium zusätzlich inerte Lösungsmittel eingesetzt werden.

9. Verfahren nach Anspruch 8, dadurch gekennzeichnet, daß die inerten Lösungsmittel bei Raumtemperatur von etwa 20°C flüssig sind.

10. Verfahren nach Anspruch 8 oder 9, dadurch gekennzeichnet, daß als inerte Lösungsmittel n-Hexan und/oder Toluol eingesetzt werden.

11. Verfahren nach mindestens einem der vorhergehenden Ansprüche, dadurch gekennzeichnet, daß als Kohlenhydrate Saccharose, Stärke oder Cellulose eingesetzt werden.

12. Verwendung einer nach mindestens einem der vorhergehenden Ansprüche hergestellten silylierten Cellulose zur Herstellung von Regenerat-Cellulosefasern nach dem Schmelz- oder Naßspinnverfahren.

13. Verwendung einer nach mindestens einem der vorhergehenden Ansprüche hergestellten silylierten Cellulose zur weitergehenden Umsetzung mit Acylierungsmitteln.

14. Verwendung nach Anspruch 13, dadurch gekennzeichnet, daß als Acylierungsmittel ein Acetylierungsmittel eingesetzt wird.

## Claims

1. Process for the silylation of carbohydrates in liquid nitrogen compounds, characterized in that 10 parts by weight of carbohydrates are reacted under pressure in an autoclave at a temperature of 25 to 150°C with a silylating agent of the formula (I) 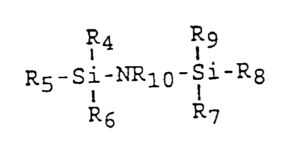 in at least 1 part by weight of a liquid nitrogen compound of the formula (II) 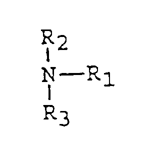 wherein, in the two formulae (I) and (II), the particular radicals, independently of one another, are:
hydrogen, a lower alkyl group having 1 to 4 carbon atoms, a cycloalkyl group having 3 to 6 carbon atoms, an aryl group in the form of a phenyl or naphthyl radical, an aralkyl group having 7 to 18 carbon atoms, an alkylaryl group having 7 to 18 carbon atoms or an O-, S- or N-containing heterocyclic group having 2 to 5 carbon atoms.

2. Process according to claim 1, characterized in that the reaction is carried out at a temperature of 50 to 120°C.

3. Process according to claim 1 or 2, characterized in that the reaction is carried out with substantial exclusion of moisture.

4. Process according to claim 3, characterized in that the exclusion of moisture is achieved by prior adequate drying of the reaction partners.

5. Process according to at least one of the preceding claims, characterized in that in the formula (II) of the nitrogen compound, the radicals R₁, R₂ or R₃ are a methyl group and/or hydrogen.

6. Process according to claim 5, characterized in that ammonia is employed as the nitrogen compound of the formula (II).

7. Process according to at least one of the preceding claims, characterized in that hexamethyldisilazane is employed as the silylating agent.

8. Process according to at least one of the preceding claims, characterized in that inert solvents are additionally employed in the reaction medium.

9. Process according to claim 8, characterized in that the inert solvents are liquid at a room temperature of about 20°C.

10. Process according to claim 8 or 9, characterized in that n-hexane and/or toluene are employed as the inert solvents.

11. Process according to at least one of the preceding claims, characterized in that sucrose, starch or cellulose are employed as the carbohydrates.

12. Use of a silylated cellulose prepared according to at least one of the preceding claims for the preparation of regenerated cellulose fibres by the melt spinning or wet spinning process.

13. Use of a silylated cellulose prepared according to at least one of the preceding claims for continuing reaction with acylating agents.

14. Use according to claim 13, characterized in that an acetylating agent is employed as the acylating agent.

## Revendications

1. Procédé de silylation des hydrates de carbone dans des composés azotés liquides, caractérisé en ce que l'on fait réagir 10 parties en poids d'hydrates de carbone sous pression dans un autoclave à une température comprise entre 25 et 150°C avec un agent de silylation de formule (I) 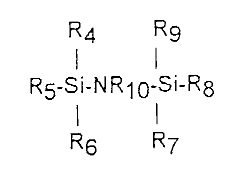 dans au moins 1 partie en poids de composé azoté liquide de formule (II) 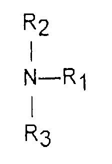 les différents radicaux, indépendants les uns des autres, représentant dans les deux formules (I) et (II) :
l'hydrogène, un groupe alkyle inférieur ayant de 1 à 4 atomes de carbone, un groupe cycloalkyle ayant de 3 à 6 atomes de carbone, un groupe aryle sous la forme d'un radical phényle ou naphtyle, un groupe aralkyle ayant de 7 à 18 atomes de carbone, un groupe alkylaryle ayant de 7 à 18 atomes de carbone ou un groupe hétérocyclique contenant O, S ou N et ayant de 2 à 5 atomes de carbone.

2. Procédé selon la revendication 1, caractérisé en ce que la réaction est effectuée à une température comprise entre 50 et 120°C.

3. Procédé selon les revendications 1 ou 2, caractérisé en ce que la réaction est effectuée à l'abri de toute humidité importante.

4. Procédé selon la revendication 3, caractérisé en ce que l'on élimine l'humidité en procédant à un séchage préalable suffisant des partenaires réactionnels.

5. Procédé selon au moins l'une des revendications précédentes, caractérisé en ce que, dans la formule (II) du composé azoté, les radicaux R₁, R₂ ou R₃ représente un groupe méthyle et/ou l'hydrogène.

6. Procédé selon la revendication 5, caractérisé en ce que l'on met en oeuvre de l'ammoniaque comme composé azoté de formule (II).

7. Procédé selon au moins l'une des revendications précédentes, caractérisé en ce que l'on met en oeuvre de l'hexaméthyldisilazane comme agent de silylation.

8. Procédé selon au moins l'une des revendications précédentes, caractérisé en ce que, dans le milieu réactionnel, on met en oeuvre, en plus, des solvants inertes.

9. Procédé selon la revendication 8, caractérisé en ce que les solvants inertes sont liquides à la température ambiante de l'ordre de 20°C.

10. Procédé selon les revendications 8 ou 9, caractérisé en ce que l'on met en oeuvre comme solvants inertes du n-hexane et/ou du toluène.

11. Procédé selon au moins l'une des revendications précédentes, caractérisé en ce que l'on met en oeuvre comme hydrates de carbone du saccharose, de l'amidon ou de la cellulose.

12. Mise en oeuvre d'une cellulose silylée, obtenue selon au moins l'une des revendications précédentes, pour fabriquer des fibres de cellulose régénérée selon le procédé de filage à chaud ou par voie humide.

13. Mise en oeuvre d'une cellulose silylée, obtenue selon au moins l'une des revendications précédentes, en vue de la faire réagir ensuite avec des agents d'acylation.

14. Mise en oeuvre selon la revendication 13, caractérisée en ce que l'on utilise comme agent d'acylation un agent d'acétylation.
